# EUROPEAN PATENT SPECIFICATION

(11) **EP 0 415 612 B1**
(45) Date of publication and mention of the grant of the patent: **10.11.1993**
(21) Application number: 90309062.9
(22) Date of filing: 17.08.1990
(51) Int. Cl.: A61K 31/135

(54) **Use of sertraline for the treatment of chemical dependencies**
Verwendung von Sertralin zur Behandlung von Abhängigkeiten von chemischen Stoffen
Utilisation de la sertraline pour le traitement des dépendances aux produits chimiques

(30) Priority: 30.08.1989 US 400647
(43) Date of publication of application: 06.03.1991
(73) Proprietor: PFIZER INC., New York, N.Y. 10017 (US)
(72) Inventor: Bick, Peter A., Hartsdale, New York (US)
(74) Representative: Bradbrook, Geoffrey William

(56) References cited:
- WO-A-90/04387
- US-A- 4 536 518
- ALCOHOL - AN INTERNATIONAL BIOMEDICAL JOURNAL, vol. 5, no. 5, September/October 1988, pages 355-358, Pergamon Press, New York, US; K. GILL et al.: "A further examination of the effects of sertraline on voluntary ethanol consumption"
- ALCOHOL - AN INTERNATIONAL BIOMEDICAL JOURNAL, vol. 5, no. 5, 1988, pages 349-354, Pergamon Press, New York, US; K. GILL et al.: "Treatment with sertraline, a new serotonin uptake inhibitor, reduces voluntary ethanol consumption in rats"
- CLINICAL NEUROPHARMACOLOGY, vol. 8, no. 4, 1985, pages 299-317, Raven Press, New York, US; L. LEMBERGER et al.: "Use of specific serotonin uptake inhibitors as antidepressants"
- PAIN, vol. 21, no. 4, April 1985, pages 329-337, Elsevier Science Publishers B.V.(Biomedical Division), Amsterdam, NL; Y.O. TAIWO et al.: "Potentiation of morphine antinociception by monoamine reuptake inhibitors in the rat spinal cord"

## Description

This invention relates to treatment of some chemical dependencies in a mammal using the compound (1S-cis)-4-(3,4-dichlorophenyl)-1,2,3,4-tetrahydro-N-methyllnaphthalenamine, hereinafter referred to by its generic name "sertraline", or a pharmaceutically acceptable salt thereof.

Sertraline, which has the empiral formula C₁₂H₁₇NCl₂ and the structural formula 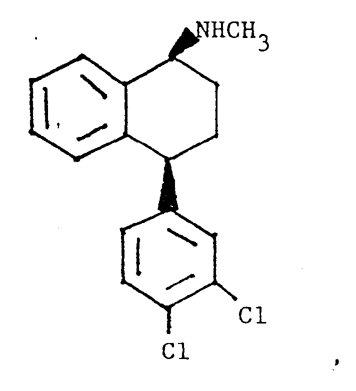
is a known antidepressant and anorectic agent.

"Alcohol - an international biomedical journal" 5(5) 349-350 and 355-380, 1988 discloses that sertraline reduces ethanol intake and ethanol preferences in rats.

The present invention relates to use of (1S-cis)-4-(3,4-dichlorophenyl)-1,2,3,4-tetrahydro-N-methyl-1-naphthalenamine, or a pharmaceutically acceptable salt thereof, for making a medicament for treating a chemical dependency on cocaine, heroin, nicotine, phenobarbital or a benzo-diazepine.

"Chemical dependency," as used herein, means an abnormal craving or desire for, or an addiction to a drug. Such drugs are generally administered to the affected individual by any of a variety of means of administration, including oral, parenteral, nasal or by inhalation.

"Treating a chemical dependency," as used herein, means reducing or alleviating such dependency.

Examples of pharmaceutically acceptable salts of sertraline that can be used to treat chemical dependencies in accordance with the present invention are the acid addition salts of various mineral and organic acids such as hydrochloric, hydrobromic, hydroiodide, sulfuric, phosphoric, acetic, lactic, maleic, fumaric, citric, tartaric, succinic, and gluconic.

Sertraline may be prepared as described in US-A-4,536,518, and particularly, in Example 2 of that patent.

Sertraline, or a pharmaceutically acceptable salt thereof, when used to treat a chemical dependency, may be administered either orally or parenterally. It is generally administered in dosages ranging from 50-200 mg per day, although variations will necessarily occur depending upon the condition of the subject being treated and the particular route of administration chosen. It may be administered either alone or in combination with pharmaceutically acceptable carriers by either of the above routes, and such administration can be carried out in both single and multiple dosages. More particularly, sertraline, or a pharmaceutically acceptable salt thereof, may be administered in a wide variety of different dosage forms, i.e., it may be combined with various pharmceutically acceptable inert carriers in the form of tablets, capsules, lozenges, troches, hand candies, powders, sprays, aqueous suspensions, injectable solutions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, sertraline, or a pharmaceutically acceptable salt thereof, when used to treat a chemical dependency, is present in such dosage forms at concentration levels ranging from 0.5% to 90% by weight of the total composition, i.e, in amounts that are sufficient to provide the desired unit dosage. It may exist in different polymorphic forms, i.e. different crystalline forms.

For purpose of oral administration, tablets containing various excipients such as sodium citrate, calcium carbonate and calcium phosphate may be employed along with various disintegrants such as starch, preferably potato or tapioca starch, alginic acid and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred fillers would also include lactose or milk sugar as well as high molecular weight polyethylene glycols. When aqueous suspensions and/or elixers are desired for oral administration, the sertraline, or pharmaceutically acceptable salt thereof, may be combined with various sweetening or flavoring agents, coloring matter or dyes and, if so desired, emulsifying and/or suspending agents, together with diluents such as water, ethanol, propylene glycol, glycerin and various like combinations thereof.

For purposes of parenteral administration, solutions of sertraline, or a pharmaceutically acceptable salt thereof, in sesame or peanut oil or in aqueous propylene glycol or N,N-dimethylformamide may be employed, as well as sterile aqueous solutions of the water-soluble, non-toxic mineral and organic acid addition salts previously enumerated. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal injection purposes. In this connection, the sterile aqueous media employed are all readily obtainable by standard techniques well-known to those skilled in the art.

A typical dry solid pharmaceutical composition is prepared by blending the following materials together in the proportions by weight specified below:
Cis-(1S)-N-methyl-4-(3,4-dichlorophenyl)-1,2,3,4-tetrahydro-1-naphthalenamine hydrochloride: 50
Sodium citrate: 25
Alginic acid: 10
Polyvinylpyrrolidone: 10
Magnesium stearate: 5
After the dried composition is thoroughly blended, tablets are punched from the resulting mixture, each tablet being of such size that it contains 100 mg of sertraline hydrochloride. Other tablets are also prepared in a similar fashion containing 5, 10, 25, and 50 mg of sertraline hydrochloride respectively, by using the appropriate amount of the naphthalenamine salt in each case.

Another typical dry solid pharmaceutical composition is prepared by combining the following materials together in the proportions by weight indicated below:
Cis-(1S)-N-methyl-4-(3,4-dichlorophenyl)-1,2,3,4-tetrahydro-1-naphthalenamine hydrochloide: 50
Calcium carbonate: 20
Polyethylene glycol, average molecular weight, 4000: 30
The dried solid mixture so prepared is then thoroughly agitated so as to obtain a powdered product that is completely uniform in every respect. Soft elastic and hard-filled gelatin capsules containing this pharmaceutical composition are then prepared, employing a sufficient quantity of material in each instance so as to provide each capsule with 50 mg of the active ingredient.

## Claims

1. Use of (1S-cis)-4-(3,4-dichlorophenyl)-1,2,3,4-tetrahydro-N-methyl-1-naphthalenamine, or a pharmaceutically acceptable salt thereof, for making a medicament for treating a chemical dependency on cocaine, heroin, nicotine, phenobarbital or a benzo-diazepine.

## Patentansprüche

1. Verwendung von (1S-cis)-4-(3,4-Dichlorphenyl)-1,2,3,4-tetrahydro-N-methyl-1-naphthalinamin oder eines pharmazeutisch verträglichen Salzes davon zur Herstellung eines Arzneimittels zur Behandlung einer Chemikalienabhängigkeit von Kokain, Heroin, Nikotin, Phenobarbital oder Benzo-diazepin.

## Revendications

1. Utilisation de (1S-cis)-4-(3,4-dichlorophényl)-1,2,3,4-tétrahydro-N-méthyl-1-naphtalénamine, ou d'un de ses sels pharmaceutiquement acceptables, pour la préparation d'un médicament destiné au traitement d'une dépendance chimique vis-à-vis de la cocaïne, de l'héroïne, de la nicotine, du phénobarbital ou d'une benzodiazépine.
